# FASCICULE DE BREVET EUROPEEN

(11) **EP 3 003 171 B1**
(45) Date de publication et mention de la délivrance du brevet: **14.11.2018**
(21) Numéro de dépôt: 14803818.5
(22) Date de dépôt: 30.05.2014
(51) Int. Cl.: A61B 17/12, A61B 17/42, A61M 31/00

(54) **DISPOSITIF D'HEMOSTASE**
HÄMOSTASEVORRICHTUNG
HAEMOSTASIS DEVICE

(30) Priorité: 30.05.2013 FR 1354941
(43) Date de publication de la demande: 13.04.2016
(73) Titulaire: Daoud, Mamia, 92290 Chatenay Malabry (FR); Guergah, Selmene, 75014 Paris (FR); Daoud, Faissal, 21300 Chenove (FR)
(72) Inventeur: Daoud, Mamia, 92290 Chatenay Malabry (FR); Guergah, Selmene, 75014 Paris (FR); Daoud, Faissal, 21300 Chenove (FR)
(74) Mandataire: Cabinet Chaillot
(86) Numéro de dépôt international: PCT/FR2014/051288
(87) Numéro de publication internationale: WO 2014/191702

(56) Documents cités:
- WO-A1-98/47558
- WO-A1-99/29363
- WO-A2-2008/100433
- WO-A2-2012/058289
- DE-U1-202011 003 097
- US-A- 4 976 692
- US-A- 5 256 141
- US-A1- 2001 053 920
- US-A1- 2009 204 099
- US-A1- 2011 094 655
- US-B1- 6 726 651

## Description

La présente invention concerne un dispositif d'hémostase utilisable en cas d'hémorragie du post partum et ses applications.

L'hémorragie du post partum (HPP) est responsable de la mort de plus de 125000 parturientes par an dans le monde et 14 millions de femmes en sont victimes chaque année selon l'OMS.

En France l'hémorragie de la délivrance touche 5% de parturientes et les cas graves représentent près de 1,7%. Plus de la moitié des morts maternelles par HPP sont évitables d'après une récente étude multicentrique.

Cette hémorragie, estimée sur les 24 premières heures, est définie comme étant une perte de plus de 500 ml de sang. L'hémorragie grave est définie comme une perte de plus de 1 litre de sang ou 4 g d'hémoglobine (convenue par les sociétés savantes nationales et internationales : en France CNGOF et SFAR ; en Grande Bretagne : RCOG ; la société canadienne:SOGC et le collège américain AMCOG).

L'hémorragie du post partum est liée dans 90% des cas à une atonie utérine. Cette dernière est primaire ou inexpliquée dans 58% des cas, le reste est dû à une rétention placentaire ou des membranes. Il s'agit d'une atonie par manque de contractilité physiologique pour réaliser l'hémostase après la délivrance du placenta. En effet une rétraction utérine spontanée violente est indispensable à la ligature physiologique pour arrêter les saignements. L'hémorragie est liée à l'impossibilité du muscle utérin de se contracter efficacement et durablement pour fermer les vaisseaux materno-foetaux après la libération du placenta.

Ce lit placento-utérin contient près de 500 ml de sang qui se redistribue spontanément dans le sang maternel après la rétraction utérine normale.

Des moyens médicamenteux utérotoniques ont été préconisés pour prévenir et ou traiter le manque de contractilité utérine. Pour ce qui est de la prévention, un protocole de délivrance dirigée a été mis en place par le Collège CNGOF, qui consiste à injecter 5 à 10 unités d'ocytocine au moment du dégagement des épaules foetales, pour entraîner une délivrance sans délai et une rétraction rapide et efficace de l'utérus.

En ce qui concerne le traitement devant une atonie, après avoir éliminé une cause obstétricale, comme la rétention du placenta ou les déchirures cervico-vagino-vulvaires, un arsenal thérapeutique médicamenteux est rapidement mis en place. Dans l'ordre horaire, il commence par l'injection d'ocytocine et en cas d'échec l'injection de prostaglandine type sulprostone.

Comme autre moyen thérapeutique, on peut citer le massage utérin abdominal avec la main de l'opérateur ou encore le poids abdominal, comme l'installation d'un sac de sable ou d'un autre moyen exerçant une pression sur le muscle utérin à travers la paroi abdominale

En cas d'échec avec persistance de l'HPP, des moyens invasifs sont proposés comme l'embolisation en cas d'accouchement par voie basse ou les ligatures chirurgicales en cas de césarienne ou d'absence de plateau technique d'embolisation.

Une méthode radicale consiste à réaliser une hystérectomie en cas d'échec des méthodes conservatrices comme la chirurgie ou l'embolisation, ou d'emblée si la parturiente présente une instabilité hémodynamique.

Un autre moyen utilisé est le tamponnement intra utérin par un ballonnet.

D'après les résultats publiés (voir Doumouchtsis SK, Papageorghiou AT, Arulkumaran S. Systematic review of conservative management of postpartum haemorrhage: what to do when medical treatment fails. Obstet Gynecol Surv 2007;62:540-7), cette méthode est prometteuse, malgré l'inadaptation des ballons actuels à l'utérus . En effet des ballons de gastro entérologie (ballon de BlackMore) [Chan C, Razvi K, Tham KF, Arulkumaran S. The use of a Sengstaken-Blakemore tube to control postpartum haemorrhage. Int J Gynaecol Obstet 1997;58: 251-2] ou d'urologie (sonde de Folley ou sonde de Rusch) [Keriakos R, Mukhopadhyay A. The use of the Rusch balloon for management of severe postpartum haemorrhage. J Obstet Gynaecol 2006;26:335-8] ont été utilisés.

Des sondes de gynécologie ont été proposées comme le ballon de Bakri [Bakri YN. Balloon device for control of obstetrical bleeding. Eur J Obstet Gynecol Reprod Biol 1999;86:S33-101], mais restent d'utilisation très restreintes, car en pratique elles ne sont pas bien adaptées lors de la gestion de l'HPPI qui demande des moyens simples d'utilisation et un apprentissage rapide.

Une étude comparative multicentrique [Doumouchtsis SK, Papageorghiou AT, Arulkumaran S. Systematic review of conservative management of postpartum haemorrhage: what to do when medical treatment fails. Obstet Gynecol Surv 2007;62:540-7] entre les différentes techniques invasives type embolisation chirurgie versus non invasives comme la tamponnade (utilisation de ballons) montre 90.7% d'efficacité pour l'embolisation, et 84.0% pour le tamponnement par ballonnet, malgré la diversité des pratiques et l'utilisation de ballons peu ou pas appropriés, 91.7% pour les techniques de capitonnage, et 84.6% pour les techniques de ligatures vasculaires.

Il n'y a donc pas de preuve de supériorité d'une méthode par rapport à une autre. Les effets secondaires liés aux méthodes invasives et la non agressivité du tamponnement sont des arguments indiquant qu'il convient de privilégier le ballon quand c'est possible. C'est la raison qui a mené plusieurs sociétés savantes internationales à intégrer le ballon de tamponnade dans l'arsenal thérapeutique pour gérer l'HPP dont le collège américain, le royal collège anglais la société canadienne et la fédération internationale - la FIGO.

Il semble qu'il y ait une réticence des obstétriciens devant cette méthode de tamponnement mécanique, liée à plusieurs facteurs:
Un premier facteur consiste en la difficulté technique de pose car le col est mou et largement ouvrable et le ballon n'est maintenu par aucun moyen qui peut l'empêcher de descendre de la cavité utérine; il se propulse sous la pression du remplissage dans le vagin. Pour pallier cette difficulté, certains auteurs y ont développé des techniques associées, comme le cerclage cervical pour retenir le ballonnet en position intra utérine, ce qui rend le geste complexe avec l'agressivité du cerclage et son caractère invasif.

Un autre facteur est l'inadaptation à l'anatomie utérine qui ne permet pas de comprimer tout le fond utérin. Un espace mort subsiste pour laisser s'installer un hématome.

De plus le moyen de drainage unique fundique des ballons actuels ne permet pas un drainage efficace. Le tuyau d'évacuation se trouve comprimé entre le ballon et l'utérus. Les utilisateurs de ballons, recommandent alors de réaliser une échographie pour détecter les hématomes masqués par le ballon. Cette technique complémentaire complique la prise en charge. À ce jour toutes les salles de naissance ne sont pas équipées en échographie et encore faut-il disposer d'un échographiste et réaliser cet acte complémentaire. Or la moindre minute gagnée permet de stopper l'HPP.

Lors de césariennes, le ballon a été retenu à une suture de cloisonnement utérin type B Lynch, ce qui n'est pas sans risque de synéchie utérine.

Le risque de rupture utérine est une crainte justifiée, car lors du remplissage du ballon, la surpression dans celui-ci lutte contre une tension utérine déjà atone. Cet équilibre entre la tension pariétale utérine et la pression provoquée par le ballon est un paramètre inconnu. La quantité de liquide introduite dans le ballon reste à l'appréciation de l'opérateur et le risque de rupture est donc possible.

Avec les ballons actuels, la Pression Intra Utérine (PIU) n'est pas mesurable et donc la fragilisation de la paroi, surtout s'il y a des antécédents de cicatrice utérine, justifie cette appréhension.

Le volume à introduire dans le ballon n'est pas standardisé, et chaque opérateur décrit son propre volume de remplissage. Là également la mesure de la pression intra utérine est inconnue et donc la lutte pariétale est mise en danger par un excès de volume. L'insuffisance de remplissage par sécurité ne permet pas d'atteindre l'objectif de compression efficace.

Le ballon actuel dit "gynécologique", s'il se colle sur le corpus utérin et le comprime, ne permet pas une compression efficace sur le segment inférieur et le col. L'absence de rétraction de cette partie de l'utérus provoque les HPP les plus sévères, à cause des placentas insérés bas et de la faible composante musculaire.

Enfin la durée pour garder le ballon en place est mal définie et les différents auteurs ne sont pas d'accord sur le moment où le ballon doit être dégonflé. Selon certains cette durée devrait être de 2 heures tandis que d'autres le laissent en place 24 heures ou plus, ce qui n'est pas sans risque de thrombose veineuse.

Il serait donc souhaitable de disposer d'une sonde bien adaptée qui s'intègre dans la phase active post médicamenteuse et pré invasive pour arrêter l'HPP. Idéalement, cette sonde devrait éviter les problèmes évoqués ci-dessus.

La demande de brevet américain US 2009/0204099 A1 décrit un système de cathéter pour dilatation et maturation du col de l'utérus qui comprend un tube allongé, un premier ballon gonflable fixé à une extrémité du tube allongé, et configuré pour être placé dans la cavité utérine, un second ballon gonflable disposé entre le premier ballon gonflable et l'autre extrémité du tube allongé, et configuré pour être placé dans le col de l'utérus lorsque le premier ballon gonflable est tiré contre le col de l'utérus, et des moyens de distribution au col de l'utérus d'un médicament de maturation du col de l'utérus.

La demande de brevet américain US 2001/0007945 A1 décrit un appareil à ballon utérin dont la pression interne est réglable. Il ne comporte pas de ballon vaginal.

La demande internationale PCT WO2008/100433 A2 décrit un dispositif implantable pour contrôler une hémorragie dans une cavité corporelle. Il comporte un ballon proximal et un ballon distal disposés le long d'un conduit tubulaire, lequel traverse le ballon distal et débouche à l'extérieur de celui-ci, ce qui ne permet que le tamponnement de la partie basse de l'utérus.

La demande de brevet américain US 2006/0173486 A1 décrit un appareil pour stopper une hémorragie post-partum, comportant un seul ballon apte à venir se placer dans la cavité de l'utérus, ce qui ne permet pas de le maintenir dans la cavité.

C'est pourquoi la présente invention a pour objet un dispositif d'hémostase selon les caractéristiques de la revendication indépendante.

Les demandeurs se sont aperçus que, contrairement aux préjugés de la technique, par exemple décrite dans la demande internationale PCT WO2008/100433 A2, il ne fallait pas drainer au sommet du ballon distal, mais exercer une pression uniforme sur la paroi de la cavité corporelle traitée.

Ainsi, selon la présente invention, le fait que le conduit tubulaire ne traverse pas la paroi du ballon distal permet au ballon distal de se conformer à la forme de la cavité corporelle et par application d'une pression uniforme sur la paroi de celle-ci, de chasser le sang à la base du ballon distal. Cela permet un tamponnement total.

Le ballon proximal permet un meilleur maintien du ballon distal dans la cavité corporelle traitée, ce qui permet d'éviter à un opérateur d'avoir à maintenir le ballon distal dans la cavité corporelle.

Le ballon proximal permet également de réduire la vascularisation dans la partie haute de la cavité corporelle dans laquelle est positionné le ballon proximal.

De part la présence du ballon proximal, le sang est confiné dans un espace entre les deux ballons, permettant ainsi une hémostase optimale, ce qui n'est pas le cas des dispositifs décrits dans les demandes de brevets américains US 2001/0007945 A1 et US 2001/0007945 A1.

On évite en outre la formation de poche d'une collection sanguine au sommet du ballon distal, qui peuvent apparaître lorsque le conduit tubulaire traverse le ballon distal comme dans l'appareil décrit dans la demande internationale PCT WO2008/100433 A2.

Dans des conditions préférentielles de mise en oeuvre de l'invention, le ballon distal et le ballon proximal sont adjacents ou sont longitudinalement espacés par rapport à l'axe du conduit l'un de l'autre de 0,5 à 4,0 cm, par exemple de 0,5 à 4,0 cm, l'espacement étant fixe ou variable.

Dans des conditions préférentielles de mise en oeuvre de l'invention, le dispositif d'hémostase comprend un quatrième tube d'alimentation ou de récupération de fluide dont une partie de la longueur est installée dans le conduit tubulaire, ce quatrième tube débouchant également dans un espace extérieur au conduit, situé entre le ballon distal et le ballon proximal.

La cavité corporelle est de préférence l'ensemble de l'utérus et du vagin d'un mammifère féminin, notamment d'une femme. Dans un tel cas, le ballon distal, gonflable comme tout ballon et capable d'expansion, est dit "utérin" car il est prévu pour être installé et gonflé dans un utérus, tandis que le ballon proximal, lui aussi gonflable et capable d'expansion, est dit "vaginal" car il est prévu pour être installé et gonflé dans un vagin.

Le dispositif d'hémostase de la présente invention permet, après introduction appropriée dans la cavité corporelle à traiter, de contrôler, limiter ou stopper une hémorragie dans cette cavité corporelle, par exemple une hémorragie du post-partum. Gonflés par un fluide, le ballon distal, le ballon proximal ou les deux ballons sont capables d'exercer une force de compression contre une paroi ou l'ensemble des parois de la cavité corporelle concernée. Pour exercer cet effet, l'homme de l'art comprend qu'un ballon gonflé doit de préférence se conformer au mieux à la forme de la cavité corporelle.

En vue de son expansion, un ballon est réalisé en matériau extensible tel que le chlorure de polyvinyle, le polyéthylène, le caoutchouc naturel ou synthétique, un polyuréthane, et de manière générale tout élastomère extensible, polymérique ou non. Toutefois, dans certains cas particuliers, il peut être utile d'utiliser un matériau qui n'est pas extensible, de telle sorte que le ballon puisse conserver sa forme d'origine après gonflage.

La forme d'origine de chaque ballon, notamment du ballon distal, peut être adaptée à l'utilisation visée, de telle sorte que le ballon gonflé puisse épouser au mieux la forme de la cavité traitée. Ainsi, même sans être installé dans une cavité humaine, un ballon gonflé notamment distal, peut avoir une forme cylindrique, ovoïde, en coeur, en champignon, ou d'une goutte d'eau pendant sa chute. À cet effet, la matière d'un ballon peut notamment présenter des zones moins épaisses que d'autres, de telle sorte de que lors du gonflage l'expansion soit plus importante dans les zones les moins épaisses. Un ballon peut également comporter avant gonflage par exemple des plis, des ondulations, ou des diverticules.

Compte tenu de l'utilisation du dispositif d'hémostase de la présente invention, le matériau utilisé est avantageusement biocompatible et stérilisable, par exemple par agent antimicrobien, rayonnement gamma ou autres techniques de stérilisation.

L'axe du ballon distal allongé est son grand axe. Si le ballon proximal est également allongé, il en est de même. Par contre si le ballon proximal a une forme annulaire et notamment une forme torique, l'axe auquel on se réfère est l'axe de symétrie ou pseudo symétrie du ballon, passant par son centre.

L'ouverture du ballon distal est avantageusement installée autour de l'extrémité distale du conduit tubulaire. De préférence, le ballon distal comprend une seule ouverture, qui est celle qui permet son installation à l'extrémité distale du conduit tubulaire.

L'homme de l'art comprend également que le conduit tubulaire, en vue de son introduction, est de préférence rigide ou semi rigide, de telle sorte que le ballon distal puisse être installé sans difficulté majeure dans la cavité corporelle à traiter, par exemple au niveau de l'utérus d'une femme.

Ce tube comprend avantageusement un repère visible prévu 1 à 2 cm de l'extrémité distale du ballon utérin. L'utilité de ce repère, qui devrait alors rester visible dans le vagin à la limite de la sortie du col, sera expliquée ci-après.

Le conduit tubulaire pourra avoir une longueur avantageusement de 06 à 60 cm, de préférence de 06 à 55 cm, notamment de 08 à 50 cm, particulièrement de 10 à 45 cm, tout particulièrement de 10 à 40 cm. Il pourra avoir un diamètre externe avantageusement de 0,1 à 10 cm, de préférence de 0,5 à 8 cm, notamment de 0,5 à 4 cm, tout particulièrement de 1 à 3 cm.

Chaque ballon est muni de son dispositif de gonflage qui comprend un tube d'alimentation ou de récupération de fluide.

L'alimentation en fluide permet le gonflage et l'expansion du ballon, tandis que la récupération de fluide permet le dégonflage partiel ou total du ballon. Il est en effet important, en cas de gonflage et donc de pression excessive, de pouvoir dégonfler le ballon jusqu'à la pression appropriée. Si un tube unique peut remplir ces deux fonctions pour chaque ballon, chaque ballon peut avoir deux tubes spécialisés, l'un pour le gonflage, l'autre pour le dégonflage.

La lumière du tube permet de gonfler ou remplir l'intérieur d'un ballon avec un fluide, à savoir un gaz ou un liquide, permettant au ballon d'épouser la forme de la cavité corporelle. La lumière permet aussi de dégonfler le ballon par reflux du fluide utilisé.

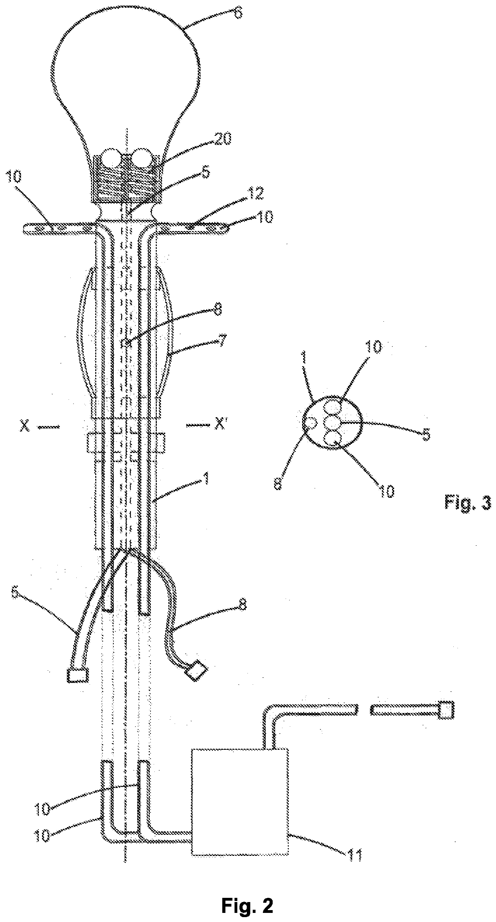

Pour éviter une surpression, notamment dans le ballon utérin, ce dernier est en communication de fluide avec au moins une soupape unidirectionnelle pour n'autoriser l'écoulement que dans le sens de la sortie du ballon distal), et réglée pour qu'une fois le ballon distal gonflé, toute surpression exercée par la paroi de la cavité corporelle traitée sur la paroi externe du ballon distal entraîne une évacuation de l'excès de fluide de gonflage par au moins une sortie. Lorsque les ballons sont gonflés, une cavité est créée entre les ballons. Une telle soupape peut alors être notamment bi fonctionnelle puisque d'une part elle permet d'éviter une surpression et d'autre part, elle peut libérer dans la cavité inter ballons le fluide utilisé pour le gonflage, ce qui permet en même temps le drainage de cette cavité dans le cas d'un fluide liquide comme on le verra ci-après sur la figure 2. Ce fluide peut alors être évacué par le tube de drainage/d'évacuation.

La ou les soupapes peuvent être installées en différents emplacements. Selon l'invention, elles sont installées à l'extrémité distale du conduit, et déboucher à l'extérieur de celui-ci. Ainsi elles peuvent libérer dans la cavité précitée le fluide utilisé pour le gonflage. Installées à l'extrémité distale du conduit, elles peuvent aussi déboucher sur un tube d'évacuation, ce qui ne fait pas partie de la présente invention. Comme on l'a vu ci-dessus, chaque ballon peut avoir deux tubes spécialisés, l'un pour le gonflage, l'autre pour le dégonflage/évacuation. Ainsi, le fluide utilisé pour le gonflage ne sera pas évacué dans l'espace entre les ballons, mais pourra être évacué à distance. La ou les soupapes peuvent aussi être installées dans le conduit tubulaire, ce qui ne fait pas partie de la présente invention non plus. Elles seront alors installées dans un tube spécialisé de dégonflage/évacuation lui-même installé dans le conduit tubulaire, dont l'extrémité distale est ouverte dans le ballon distal. La ou les soupapes peuvent aussi être installées à l'extérieur du conduit tubulaire, ce qui ne fait pas partie de la présente invention non plus. Elles seront alors installées dans ce cas aussi dans un tube spécialisé de dégonflage/évacuation lui-même installé dans le conduit tubulaire, dont l'extrémité distale est ouverte dans le ballon distal. Mais alors que dans le cas précédent la ou les soupapes sont installées dans le conduit vers l'extrémité distale du tube, dans le cas présent elles sont installées dans le tube à l'extérieur du conduit tubulaire, par exemple à une distance de 40 à 60 cm de l'extrémité distale du conduit tubulaire.

Selon l'invention, la ou les soupapes unidirectionnelles sont installées à l'extrémité distale du conduit tubulaire et autorisent le passage de l'excès de fluide vers le ou les sorties débouchant dans un espace extérieur au conduit tubulaire, situé entre le ballon distal et le ballon proximal. La ou les soupapes unidirectionnelles peuvent être disposées à l'extrémité proximale d'un ou de tubes de sortie, l'extrémité distale desdits tubes de sortie débouchant à la base du ballon distal ce qui ne fait pas partie de la présente invention.

La ou les soupapes pourront être des soupapes automatiques, dont le seuil de déclenchement peut être réglé de 00 à 200 mm de mercure, avantageusement de 20 à 180 mm de mercure, de préférence de 30 à 130 mm de mercure, notamment de 30 à 120 mm de mercure. La ou les soupapes pourront être aussi des soupapes à commande manuelle, contrôlées par un opérateur. Dans ce dernier cas, elles seront avantageusement installées à l'extérieur du conduit tubulaire.

L' un des trois tubes fondamentaux peut être en communication avec au moins un orifice d'aspiration pour récupérer puis évacuer le liquide formé dans l'espace entre les deux ballons distal et proximal gonflés et déboucher dans un espace extérieur au conduit, situé entre le ballon distal et le ballon proximal. Ce tube, appelé tube d'évacuation, sert au drainage ou à la vidange de cette cavité. Ce tube de drainage/d'évacuation sert à évacuer notamment le sang hémorragique. La zone où débouche ce tube sera alors prévue par exemple de 0.5 à 10 cm, avantageusement de 0.5 à 8 cm, de préférence de 0.5 à 4 cm, notamment de 1 à 2 cm de l'extrémité distale du conduit tubulaire. Ce tube se prolongera avantageusement à l'extérieur du conduit tubulaire pour former une sorte de tentacule dont la longueur pourra aller jusqu'à quelques centimètres. La longueur de cette tentacule pourra aller par exemple de 1 mm à 8 cm, de préférence de 5 mm à 7 cm, particulièrement de 1 cm à 7 cm, plus particulièrement de 2 cm à 6 cm, tout particulièrement de 3 cm à 6 cm.

Le ou les orifices d'aspiration peuvent être l'embouchure du troisième tube qui affleure la paroi externe du conduit tubulaire (1), l'embouchure du troisième tube en saillie de la paroi externe du conduit tubulaire (1), ou des orifices formés sur une partie du troisième tube en saillie du conduit tubulaire (1) formant une tubulure multipores

Dans un mode de réalisation de l'invention, on trouve un quatrième tube débouchant également dans un espace extérieur au conduit, situé entre le ballon distal et le ballon proximal. Ce tube peut alors servir à l'injection d'un fluide dans la cavité, encore appelé tube d'irrigation. Ainsi, deux fluides différents peuvent être utilisés, l'un pour le gonflage du ballon distal et l'autre pour apporter du liquide de drainage.

La réalisation du conduit et de l'ensemble de tubes peut prendre diverses formes. On peut par exemple avoir un conduit tubulaire dans lequel sont installés les trois, (ou selon le cas quatre) tubes fondamentaux, étant rappelé que chacun des tubes peut-être dédoublé par exemple pour en scinder les fonctions (par exemple deux tubes pour chaque ballon, deux tube d'irrigation, deux tube de drainage etc.). On peut aussi prévoir un conduit, par exemple extrudé, comprenant trois, quatre canaux, ou plus correspondant à une partie des tubes ci-dessus ou à tous les tubes ci-dessus.

À titre illustratif, les ouvertures des lumières des canaux situés à la partie proximale du conduit peuvent être munies de connecteurs comme des cônes Luer, Luer lock ou autres, pour connexion à des tuyaux flexibles. L'ensemble des tuyaux et canaux correspond alors aux tubes ci-dessus. On peut évidemment faire un mélange des solutions ci-dessus, par exemple le conduit peut comprendre trois canaux dont un canal sert de passage à deux tubes, tandis que les deux autres canaux constituent une partie de deux autres tubes fondamentaux.

Dans les modes de réalisation mettant en oeuvre un conduit comprenant des canaux, ceux-ci débouchent par une ouverture prévue à l'endroit approprié.

Lorsque les tubes sont indépendants du conduit, ils peuvent être réalisés dans les mêmes matériaux que ceux utilisés pour les ballons. Ils sont de préférence réalisés en polyuréthane, silicone, polychlorure de vinyle ou autres élastomères.

L'épaisseur des tubes est adaptée à leurs fonctions. Il est clair pour l'homme de l'art que lorsque l'on procède à un gonflage, c'est le ballon qui doit se gonfler et non le tube correspondant.

De préférence, les tubes débouchant dans l'espace extérieur au conduit, situé entre le ballon distal et le ballon proximal débouchent dans une portion distale du conduit située entre 2 mm et 5 cm de l'extrémité distale du conduit. Si les ouvertures peuvent déboucher directement au niveau du conduit, avantageusement, une courte tubulure, par exemple de 5 mm à 5 cm, de préférence de 1 à 10 cm, notamment de 2 à 8 cm, tout particulièrement de 1 cm à 7 cm, s'étend à partir du conduit pour écarter les ouvertures de celui-ci. Dans un tel cas, les tubulures sont avantageusement inclinées vers l'extrémité distale du conduit et donc vers le ballon distal.

Les canaux et tubes ont un diamètre intérieur adapté à leur fonction. Ceux prévus pour l'injection et le drainage extérieur au dispositif ont habituellement un diamètre supérieur à ceux prévus pour le gonflage et dégonflage des ballons, notamment pour éviter d'être bouché par des débris ou caillots sanguins. Également, les dispositifs d'hémostase de l'invention peuvent être des dispositifs d'urgence. L'urgence pouvant être de stopper une hémorragie, il convient de pouvoir rapidement gonfler les ballons, notamment le ballon distal. Ainsi la lumière des tubes de gonflage des ballons permet avantageusement un gonflage rapide en fonction du matériel de gonflage utilisé. Ce dernier peut être par exemple une pompe ou une seringue de diamètre de préférence important, par exemple de 3 à 5 cm de diamètre intérieur.

La même remarque que celle faite pour les tubes ci-dessus s'applique. Ainsi, tant le tube d'irrigation qui sert à l'injection d'un fluide dans la cavité, que le tube d'évacuation qui sert au drainage ou à la vidange de cette cavité, peut déboucher par une ou plusieurs ouvertures.

Les tubes sont de préférence transparents pour observer la circulation des fluides à l'intérieur de ceux-ci et notamment les éventuelles particules solides comme caillots sanguins, fragments de tissus ou autres, pouvant être véhiculé par ces fluides.

Si les orifices distaux des tubes sont habituellement simples, les orifices proximaux sont avantageusement munis de moyens de connexion tels que cônes Luer ou Luer lock, raccords, connecteurs, ou adaptateurs.

Les tubes peuvent être munis du côté distal d'une ou plusieurs dérivations, par exemple en Y.

Certains tubes peuvent être également munis de dispositifs tels que des soupapes unidirectionnelles pour n'autoriser l'écoulement que dans un sens. Il en est de même pour les ballons, comme on l'a vu ci-dessus, dans l'objectif d'éviter une surpression. Par exemple le ballon utérin est muni d'une telle soupape autorisant d'évacuation de la cavité vaginale.

Le tube de drainage/d'évacuation est avantageusement relié par son extrémité proximale à un récipient de recueil, de préférence permettant l'évaluation de la quantité de sang recueilli. À cette fin, le récipient peut par exemple être gradué. Les conduits d'évacuation peuvent être totalement distincts ou peuvent avoir un tronc commun, par exemple à partir d'une zone proximale du ballon proximal.

Ce tube de drainage/d'évacuation peut-être aussi avantageusement relié par son extrémité proximale à un récipient d'observation, par exemple en matière plastique transparente, permettant par exemple d'observer la couleur du fluide évacué, ce qui permet d'évaluer notamment la présence de sang ou de matières dans le fluide évacué.

Lorsque le dispositif d'hémostase de la présente invention est muni des deux types de récipient ci-dessus, on installe de préférence le récipient d'observation entre le conduit et le récipient de recueil.

Le ou les tubes de drainage/d'évacuation pourra comprendre de préférence de 1 à 6, notamment de 1 à 4, particulièrement 1, 2 ou 3 ouvertures distales.

Dans un mode de réalisation préféré de l'invention, le dispositif d'hémostase pour une cavité corporelle comprend entre autres
- un conduit tubulaire ayant une extrémité distale et une extrémité proximale,
   - un ballon distal et un ballon proximal
   - le ballon distal et le ballon proximal étant co-axialement espacés le long du conduit, le ballon distal étant installé à l'extrémité distale du conduit tubulaire, le ballon proximal étant installé de manière étanche aux liquides autour d'une portion du conduit tubulaire,
   - trois tubes, d'alimentation ou de récupération de fluide, dont une partie de la longueur est installée dans le conduit tubulaire,
   - une soupape installée dans l'extrémité distale du conduit tubulaire pour ne permettre l'écoulement d'un fluide que dans le sens de la sortie du ballon distal, et agencée pour déverser ce fluide dans l'espace entre le ballon distal et le ballon proximal,
un des trois tubes débouchant dans le ballon distal, un autre des trois tubes débouchant dans le ballon proximal, le troisième des trois tubes débouchant dans un espace extérieur au conduit, situé entre le ballon distal et le ballon proximal.

Dans des conditions préférentielles de mise en oeuvre de ce dernier mode de réalisation, le troisième des trois tubes dépasse du conduit pour former une sorte de tentacule. Dans d'autres conditions préférentielles de mise en oeuvre de ce dernier mode de réalisation, le troisième des trois tubes est dédoublé. En d'autres termes, on met en oeuvre deux tubes répondant à la définition de "troisième des trois tubes", ces deux tubes comprenant également chacun une tentacule.

Dans un autre mode de réalisation préféré de l'invention, le dispositif d'hémostase pour une cavité corporelle comprend entre autres
- un conduit tubulaire ayant une extrémité distale et une extrémité proximale,
- un ballon distal et un ballon proximal
- le ballon distal et le ballon proximal étant co-axialement espacés le long du conduit, le ballon distal étant installé à l'extrémité distale du conduit tubulaire, le ballon proximal étant installé de manière étanche aux liquides autour d'une portion du conduit tubulaire,
- trois tubes, d'alimentation ou de récupération de fluide, dont une partie de la longueur est installée dans le conduit tubulaire, un des trois tubes débouchant dans le ballon distal, un autre des trois tubes débouchant dans le ballon proximal, le troisième des trois tubes débouchant dans un espace extérieur au conduit, situé entre le ballon distal et le ballon proximal
- un tube d'évacuation d'un fluide contenu dans le ballon distal dont une partie de la longueur est installée dans le conduit tubulaire,
- une soupape installée dans le tube d'évacuation d'un fluide contenu dans le ballon distal, soit dans la partie du tube installée dans le conduit tubulaire, soit dans la partie du tube qui n'est pas installée dans le conduit tubulaire,

Dans des conditions préférentielles de mise en oeuvre de ce dernier mode de réalisation, le troisième des trois tubes dépasse du conduit pour former une sorte de tentacule. Dans d'autres conditions préférentielles de mise en oeuvre de ce dernier mode de réalisation, le troisième des trois tubes est dédoublé. En d'autres termes, on met en oeuvre deux tubes répondant à la définition de "troisième des trois tubes", ces deux tubes comprenant également chacun une tentacule.

Le débit sanguin procure également des informations intéressantes. C'est pourquoi dans des conditions préférentielles de mise en oeuvre de l'invention, un débitmètre est installé sur le conduit d'évacuation pour mesurer par exemple le débit instantané de sang est ainsi orienter un diagnostic d'HPP ou évaluer l'efficacité du tamponnement (arrêt ou réduction du saignement).

La longueur du tube d'évacuation est adaptée à sa fonction d'évacuation du sang hémorragique depuis un utérus. Ainsi sa longueur pourra aller de 40 cm à 1 m, de préférence de 45 cm à 80 cm, particulièrement de 50 cm à 70 cm. Son diamètre interne pourra être constant ou variable, par exemple progressif au fur et à mesure que l'on se rapproche du côté proximal. Un diamètre interne moyen pourra être par exemple de 1 millimètre à 20 millimètres, de préférence de 10 millimètres à 15 millimètres. Le fait de parler de diamètre n'impose pas une section circulaire du conduit d'évacuation, même si un conduit cylindrique est préféré.

Il est à noter que dans la présente demande, classiquement l'article indéfini "un" doit être considéré comme un pluriel générique (signification de "au moins un" ou encore "un ou plusieurs"), sauf lorsque le contexte montre le contraire (1 ou "un seul"). Ainsi, par exemple, lorsque l'on dit ci-dessus que chaque ballon comprend un tube de gonflage/dégonflage, il convient de comprendre que chaque ballon comprend un ou plusieurs tels tubes. L'homme de l'art comprend en effet que chacun des tubes peut être prévu en double, voire en triple. De même, un même tube peut comprendre plusieurs ouvertures distales.

Les tubes de gonflage/dégonflage de chacun des ballons pourront comprendre de préférence de 1 à 6, notamment de 1 à 4, particulièrement 1, 2 ou 3 ouvertures distales. Lorsque plusieurs ouvertures distales sont prévues, celles-ci sont de préférence longitudinalement espacées. Par exemple dans le cas de trois ouvertures distales pour un tube donné, on en trouvera une à l'extrémité distale du ballon, une en position médiane, et une en position plus proximale.

Les deux ballons peuvent être adjacents. Toutefois dans des conditions préférentielles de réalisation, ils sont espacés l'un de l'autre de 0,1 à 5 cm, de préférence de 0,2 à 4,5 cm, notamment de 0,5 à 4,0 cm, particulièrement de 2,0 à 4,0 cm. Un tel espacement permet de prendre en compte l'anatomie du col de l'utérus.

L'homme de l'art comprendra qu'en vue notamment du remplissage, le tube de gonflage des ballons comprend un orifice proximal et un orifice distal, ce dernier débouchant dans le ballon. Du côté proximal, ce conduit peut être relié, comme on l'a vu ci-dessus, à un dispositif de gonflage comme une seringue ou un gonfleur. Dans ce but, le conduit peut-être muni d'un port d'entrée comme par exemple un cône Luer lock femelle ou autre adaptation par exemple vissante ou à baïonnette.

Le gonflage des ballons pourra se faire à l'aide d'un fluide gazeux ou liquide, de préférence à l'aide d'un liquide, notamment stérile. On peut utiliser notamment de l'eau ou une solution saline à 9 pour 1000. On peut également additionner le liquide utilisé pour le drainage avec notamment un ou plusieurs principes actifs pharmaceutiques. En particulier dans le cas d'un liquide notamment stérile, on peut se dispenser d'un quatrième tube d'alimentation ou de récupération de fluide débouchant dans l'espace extérieur au conduit, situé entre le ballon distal et le ballon proximal.

Le ballon distal pourra être généralement rempli par un volume allant jusqu'à 1 litre de fluide, mais dans des conditions opératoires normales, habituellement il sera rempli jusqu'à 500 ml de fluide. Dans certaines conditions, le volume pourra aller de 100 à 3500 ml de fluide.

Il est important d'éviter une surpression lors du gonflage des ballons. C'est pourquoi, dans des conditions préférentielles de mise en oeuvre de l'invention, le dispositif d'hémostase ci-dessus comprend en outre un dispositif de mesure de la pression dans le ballon distal, dans le ballon proximal, ou de préférence dans chacun des deux ballons. Ainsi, un capteur manométrique peut être installé dans chacun des deux ballons, par exemple en l'insérant par le conduit de remplissage, ou peut être installé également directement sur les tubes de gonflage/dégonflage.

Dans des conditions préférentielles de mise en oeuvre, notamment pour les dispositifs d'hémostase prévue pour les cavités utérines et vaginales, le ballon distal présente une double paroi. Alors avantageusement un supplément de coussinets est prévu pour renforcer la pression de tamponnage dans le secteur du lit placentaire. Ces coussinets peuvent être disposés pour correspondre au fond utérin, aux parois latérales et au segment inférieur.

Dans d'autres conditions préférentielles de mise en oeuvre de l'invention, la paroi externe du ballon utérin est réalisée en un matériau absorbant tel qu'une gaze. L'utilisation d'un tel matériau aide à obtenir un drainage homogène et évite la formation de caillots.

Lors d'un gonflage sans contrainte, par exemple à l'air libre, le ballon distal prendra avantageusement une forme cylindrique, notamment ovoïde. D'autres formes comme une forme en coeur, en champignon ou goutte d'eau sont également possibles. Pour sa part, le ballon proximal pourra être moins allongé et pourra même prendre une forme torique. Lorsqu'il est creux en son milieu, par exemple lorsqu'il est de forme torique, ce qui est une forme de réalisation préférée, le creux permet le passage par exemple du tube de récupération du sang hémorragique depuis un utérus, en passant à l'extérieur du conduit. Ce tube de récupération pourra cependant passer contre la périphérie du ballon proximal.

Lors d'un gonflage sans contrainte, par exemple à l'air libre, le ballon distal prendra avantageusement une forme ovoïde.

Un anneau de fixation ou analogue est avantageusement installé coulissant à l'extérieur de la partie proximale du conduit tubulaire afin de fixer le dispositif à une ceinture ou sangle abdominale.

Avantageusement, le conduit est muni de séries de crans dans lesquels par exemple un anneau ou tube extérieur peut se bloquer dans une position choisie par l'utilisateur. L'intérêt de ces crans et de l'anneau ou analogue sera expliqué ci-après.

Dans des conditions tout à fait préférentielles de mise en oeuvre de l'invention, le dispositif d'hémostase ci-dessus comprend en outre des moyens vibratoires, ou des moyens d'électrostimulation utérine, ou les deux, installés dans le ballon distal.

Les moyens d'électrostimulation utérine comprennent des câbles électriques cheminant le long du dispositif d'hémostase, à l'extrémité distale desquels sont prévues des électrodes stimulantes jouant un rôle de pacemaker vis-à-vis de l'utérus. Ainsi, l'utérus peut reprendre son activité physiologique. En coopération avec le gonflage ci-dessus, on peut réduire ce dernier, éventuellement d'une façon automatique, dès que le tonus de base d'hémostase est obtenu et que le débit de sang perdu devient insignifiant.

Des moyens vibratoires contribuent à la restauration du tonus utérin, mais procurent des résultats moindres que ceux obtenus par électrostimulation.

Ces câbles sont destinés à être reliés à un générateur réglable par exemple en voltage, intensité et fréquence pour adapter les moyens mis en oeuvre à la situation clinique. Les câbles peuvent alors chacun comprendre à leur extrémité proximale un connecteur pour connexion à un tel générateur.

Les dispositifs d'hémostase objets de la présente invention possèdent de très intéressantes qualités.

Par exemple dans une application obstétricale, les deux ballons agissent par compression du lit vasculaire de la décidue post délivrance et surtout à l'emplacement du placenta. Cette compression par dilatation de la cavité peut entraîner une contractilité utérine réactive.

La compression par tamponnement également entraîne une compression vasculaire des artères utérines et ainsi réduit le flux sanguin utérin et aboutit à l'hémostase.

Les dispositifs d'hémostase objets de la présente invention permettent de pallier la difficulté technique des ballonnets de l'art antérieur puisque la pose est rendue facile grâce à leur conception qui guide les ballons et permet de les maintenir exactement au contact avec le fond utérin grâce notamment à la rigidité de son axe et lorsqu'ils sont présents, aux repères anatomiques à sa surface. En outre, le système de fixation permet un maintien autonome libérant l'opérateur de manoeuvres inutiles.

La courbe d'apprentissage des praticiens est rapide vu qu'il s'agit de gestes de pose de Dispositif Intra Utérin (DIU) similaires à ceux pratiqués dans la vie professionnelle quotidienne d'un gynécologue obstétricien.

Les ballons des dispositifs d'hémostase objets de la présente invention se moulent sur la cavité féminine en s'adaptant à l'anatomie utéro-vaginale. Lorsque les deux ballonnets sont gonflés, il n'y a plus besoin de tenir le dispositif de l'invention. Ils se dégonflent et se regonflent quand l'utérus se contracte en s'accordant avec la PIU.

Les ballons des dispositifs d'hémostase objets de la présente invention permettent une compression efficace y compris dans le segment inférieur de l'utérus, ce qui réduit le flux sanguin dans les artères vaginales et utérines. Ils compriment un éventuel lit hémorragique de placenta bas inséré ou d'atonie du segment inférieur. Au fond utérin, ils évitent la formation d'hématomes. Les deux ballonnets délimitent une cavité intermédiaire dans laquelle le sang provenant de l'utérus, ainsi que l'eau provenant du ballon distale via la ou les soupapes ou du quatrième tube. Enfin cet espace contient également un ou des tubes de drainage

Le dispositif de l'invention, lorsqu'il est associé à un flacon collecteur, à un débitmètre, ou aux deux, permet une quantification exhaustive des pertes dès la mise en place du dispositif.

Lorsque plusieurs niveaux d'évacuation sont prévus, le drainage étagé multiple, réalisé à différents endroits des cavités utéro-vaginales, évite la formation d'hématomes localisés.

Le drainage peut-être augmenté par utilisation de matière absorbante pour réaliser tout ou partie de la surface externe du ballon utérin. Un rainurage prévu entre des coussinets placés à la surface du ballon peut aussi aider au drainage.

En raison de la conception simple du dispositif de l'invention, un système de lavage par injection d'un fluide dans un tuyau bouché ou au contraire par aspiration selon le cas permet un débouchage facile si besoin est.

Comme le dispositif permet de faire cesser l'HPP dans des proportions très importantes, le dispositif de l'invention permet d'éviter un transfert inutile de la patiente ou permettre un transfert sécurisé en accompagnant la patiente munie de son dispositif entre deux structures hospitalières.

Son efficacité permet d'éviter une transfusion sanguine ou de réduire la quantité de Produits Sanguins Labiles (PSL) à transfuser

Il permet aussi d'éviter ou de réduire les séjours en réanimation et de ne pas séparer la mère et l'enfant

Le dispositif de l'invention permet également d'éviter les gestes invasifs et permet également de traiter à égale chance les parturientes quel que soit le niveau de maternité, le ballon permettant de réduire les pertes pendant le transfert.

Une consistance semi rigide ou rigide de la sonde permet, une fois le col repéré par les valves vaginales, une pose simple comme un stérilet, sans aucune difficulté. Un obstétricien peut guider la sonde et la placer manuellement dans l'utérus.

En outre, lorsqu'ils sont munis de moyens vibratoires ou de moyens d'électrostimulation, ils permettent de relancer la contractilité utérine défaillante lors de l'atonie. Cette stimulation permet de renforcer et déclencher les Pacemakers à plusieurs endroits du corps et du col utérin, et donc lui permettre de reprendre son activité physiologique. Par conséquent, le gonflage est réduit proportionnellement, éventuellement d'une façon automatique et dès que le tonus de base d'hémostase est requis et que le débit de sang perdu est insignifiant, alors le dispositif peut alors être retiré progressivement. Cela simplifie le protocole et évite la compression prolongée avec un ballon. Cela peut aussi éviter les thromboses veineuses décrites avec les ballons de l'art antérieur.

Le gonflage peut être réalisé grâce à la détermination d'une pression, d'une tension pariétale et en fonction du rayon de l'utérus et de l'épaisseur de la paroi utérine.

Le dispositif d'hémostase de la présente invention n'empêche pas la diminution du rayon de l'utérus pour réaliser l'hémostase. La contraction musculaire peut-être suscitée de manière externe par massage de l'opérateur ou interne par le ballon, par la vibration ou l'électrostimulation.

La contraction utérine sur le ballon vient obstruer les vaisseaux ouverts et la paroi utérine vient se mettre au contact avec le ballon. L'utérus sous la compression du ballon réajuste son volume et vide le ballon. L'utérus se rétracte alors et s'épaissit.

Éventuellement, la vibration ou l'électrostimulation va augmenter la PIU, le ballon se dégonflera tout en maintenant la compression des vaisseaux sanguin. A contrario lorsque le muscle se relâche la PIU chute et le ballon gonfle pour maintenir la PIU constante.

L'efficacité du dispositif de l'invention est mesurable par la quantification de la qualité contractile de l'utérus et le débit du sang perdu. Le présent dispositif d'hémostase permet d'agir sur un temps court, avec un maximum d'efficacité.

Le dispositif de l'invention peut être contrôlé, pour certaines de ses fonctions, par utilisation d'un ordinateur. Par exemple, le débitmètre optionnel peut être relié à ce dernier, qui peut déclencher une alarme lorsque que la perte de sang est trop rapide par rapport à une valeur de référence. Également l'ordinateur peut contrôler la pression de gonflage des ballons. Dans les versions les plus élaborés, l'ordinateur peut également contrôler des vibrations (vibrostimulation) ou une électrostimulation, lesquelles sont destinées à rétablir ou renforcer la tonicité utérine.

Dans le cas d'une électrostimulation ou d'une vibrostimulation, le dispositif de l'invention comporte au moins une soupape et la paroi externe du ballon distal comporte au moins une partie conductrice, laquelle est connectée à un fil conducteur d'alimentation en courant, lequel fil conducteur passe avantageusement dans le conduit tubulaire, la pression de rupture de la ou des soupapes étant inférieure à la pression de l'organe dans lequel se trouve le ballon distal. La demande de brevet américain US 2013/0261702 A1 décrit un ballon avec de telles parties conductrices.

À cette fin, les paramètres spécifiques de la patiente peuvent être introduits préalablement dans l'ordinateur pour mieux contrôler les réglages d'utilisation du dispositif d'hémostase de l'invention.

On peut notamment utiliser simultanément ou séparément les techniques de tamponnade, de vibration et d'électrostimulation.

Néanmoins, la combinaison de ces éléments peut faciliter l'hémostase en permettant à la fois la contraction utérine par électrostimulation tout en tamponnant la paroi, le résultat étant un accompagnement de l'hémostase par cycles synchronisés de gonflement dégonflement et de stimulis électriques et/ou vibratoires. Ceci est rendu possible lorsque la pression d'ouverture de la soupape est inférieure à la pression développée par la pression utérine. Le ballon se dégonfle alors lorsque l'utérus se contracte et se regonfle lors de sa décontraction, au fur et à mesure le volume utérin se réduit et l'hémostase est réalisée. Il est à noter que pour avoir des contractions utérines suffisantes en nombre et en intensité ses stimulis électriques et/ou vibratoires nécessiteraient l'administration d'utérotoniques tel que l'ocytocine ou Sulprostone.

Le fonctionnement est personnalisable en fonction des antécédents de la patiente, de sa grossesse et de son accouchement. L'utilisation d'un capteur de pression intra utérine permet notamment grâce à l'ordinateur, de moduler le gonflage / dégonflage du ballon utérin en fonction de la tolérance utérine et de l'efficacité du tamponnement.

Un dispositif d'hémostase de l'invention peut notamment être utilisé comme suit chez une femme :
La description qui suit est donnée avec une version élaborée de la présente invention, comportant notamment une soupape. Certaines étapes, comme l'électrostimulation par exemple, sont donc optionnelles.

On procède tout d'abord à une série de mesures initiales.

Hystéromètrie : mesure la hauteur totale de la cavité utérine du fond au col, après pose de valves vaginales pour apprécier la taille de l'utérus.

Mesure de la longueur totale allant du col à la sortie du vagin. Cette longueur peut être utile dans certaines conditions d'insertion du dispositif, elle est modifiable également par rapprochement ou éloignement du col utérin qui est mobilisable.

Le dispositif de l'invention est inséré au niveau du segment inférieur de l'utérus ; de préférence à sa surface un repère visible indique la bonne hauteur afin de laisser la partie inter ballons du dispositif dans le vagin. Ce repère reste à la limite externe apparente du col. Le gonflage du ballon utérin (distal) peut commencer à ce moment là afin de fixer le dispositif au juste emplacement. Le ballon vaginal (proximal) est ensuite gonflé, les valves vaginales retirées et l'extrémité proximale du dispositif est alors de préférence fixée à une ceinture externe. Le col utérin est pris en sandwich entre les deux ballons.

Une ceinture optionnelle est mise en place en début d'installation et a une double fonction. Elle prend une hauteur suffisante de la sangle abdominale, lorsqu'elle est serrée et elle réduit le volume de la cavité pelvienne et abdominale afin de ne laisser qu'un petit volume à l'expansion utérine. Elle contribue ainsi à la réduction du volume de remplissage utérin par tamponnement externe indirect. Sa deuxième fonction est de permettre de fixer le dispositif de l'invention à sa partie proximale et le rendre autonome, libérant ainsi la main de l'opérateur.

Le ballon utérin est gonflé avec un liquide physiologique comme du sérum physiologique, délivré de préférence avec un débit constant de façon automatique (pompe, seringue, garrot électrique...). Dès que la limite de pression est atteinte, l'excès du liquide est évacué par la ou les soupapes contrôlant ainsi la pression intra-utérine (PIU).

Le ballon vaginal est gonflé, bloque les liquides dans la cavité inter-ballons pour évacuation. Le volume administré pour gonfler le ballon utérin varie selon le conduit anatomique et il est habituellement entre 300 et 800 ml. Il permet de créer un espace clos (inter-ballons) entre le col et le haut vagin. La compression exercée par ce ballon permet de baisser la circulation sanguine cervico-vaginale. En effet cet espace est en rapport avec les artères cervico-vaginales, qui perfusent cette zone, et sont inaccessibles aux ligatures et ou à l'embolisation, ce qui explique un certain échec de ces techniques.

Le système de lavage/évacuation dans l'espace inter-ballons permet de récupérer le liquide provenant du ballon utérin libéré par la ou les soupapes, diluant le sang évitant ainsi la formation de caillots. Ce système récupère également le sang provenant de l'utérus et/ou du haut vagin, et évacue ces liquides par l'intermédiaire de un ou plusieurs tubes de drainage partant de cet espace et se terminant par exemple dans un sac ou récipient de recueil.

L'efficacité du tamponnement est jugée par la clarté du liquide drainé. La surveillance de ce dernier est importante au cours de la demi-heure qui suit l'intervention.

C'est pourquoi la présente demande a aussi pour objet l'utilisation des dispositifs d'hémostase ci-dessus décrits, dans le contrôle de l'hémorragie du post-partum.

La présente demande décrit également un procédé de contrôle de l'hémorragie du post-partum comprenant les étapes consistant à
- introduire par son extrémité distale un ensemble de ballons comprenant un ballon allongé distal gonflable et un ballon proximal gonflable dans les cavités respectives du vagin et de l'utérus,
- à gonfler le ballon distal pour appliquer une pression à l'utérus, et
- à gonfler le ballon allongé proximal de telle sorte que le col utérin se trouve coincé dans une cavité entre les deux ballons.

Dans des conditions préférentielles de mise en oeuvre, le procédé ci-dessus décrit comprend en outre l'étape consistant à évacuer et de préférence recueillir le sang éventuellement présent dans l'utérus ou dans l'utérus et dans le vagin, grâce à un conduit d'évacuation muni d'une ou plusieurs ouvertures distales.

Dans d'autres conditions préférentielles de mise en oeuvre du procédé ci-dessus décrit, on mesure le débit sanguin du conduit d'évacuation, ou on mesure la quantité de sang recueilli, ou de préférence on procède à ces deux mesures.

Dans encore d'autres conditions préférentielles de mise en oeuvre du procédé ci-dessus décrit, on contrôle la pression de gonflage du ballon distal ou de préférence la pression de gonflage des deux ballons.

Les conditions préférentielles de mise en oeuvre des dispositifs d'hémostase ci-dessus décrites s'appliquent également aux autres objets de l'invention visés ci-dessus, notamment aux procédés pour leur utilisation.

L'invention sera mieux comprise si l'on se réfère aux dessins annexés sur lesquels
- la figure 1 représente une vue en coupe partielle d'un dispositif d'hémostase ne faisant pas partie de la présente invention
- la figure 2 représente une vue en coupe partielle d'un mode de réalisation du dispositif d'hémostase selon l'invention, muni d'une soupape distale,
- la figure 3 représente une vue en coupe selon X-X' de la figure 2.
- la figure 4 représente une vue en coupe partielle d'une variante du dispositif d'hémostase ne faisant pas partie de la présente invention, muni d'une soupape distale différemment placée.
- la figure 5 représente une vue en coupe selon A-A' de la figure 4.

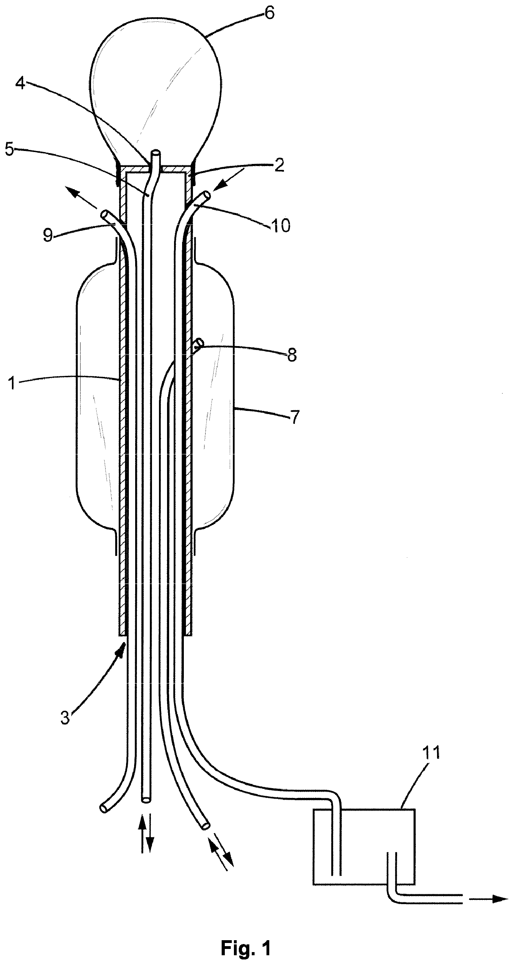

Sur la figure 1, on peut observer un conduit tubulaire 1 dont l'extrémité proximale 3 est ouverte tandis que l'extrémité distale 2 est fermée à l'exception d'un orifice 4 permettant à un tube 5 de déboucher dans un ballon distal 6 qui vient coiffer l'extrémité distale 2 du conduit tubulaire 1. Ce ballon distal 6 est installé de manière étanche à l'extrémité distale 2 du conduit tubulaire 1.

Un autre ballon, nommé ballon proximal 7 est installé de manière étanche autour de la partie médiane du conduit tubulaire 1.

Quatre tubes (où cathéters) réalisés en polyuréthane cheminent dans le conduit tubulaire 1. Une partie de la longueur de ces tubes est installée dans le conduit tubulaire 1 tandis qu'une autre partie de leur longueur est installée à l'extérieur du conduit tubulaire 1, en sortant par son extrémité proximale 3 ouverte.

Un des tubes 5 débouche dans le ballon distal 6. Ce tube 5 peut servir aussi bien au gonflage qu'au dégonflage du ballon distal 6. Un autre tube 8 débouche dans le ballon proximal 7. Ce tube 8 peut servir aussi bien au gonflage qu'au dégonflage du ballon proximal 7. Un autre tube 9 sort du conduit tubulaire 1 à proximité de son extrémité distale 2. Ce tube 9 pourra servir à irriguer la zone où il débouche. Un autre tube 10 sort du conduit tubulaire 1 à proximité de son extrémité distale 2. Ce tube 10 pourra servir à drainer la zone où il débouche par exemple en évacuant un liquide d'irrigation arrivé par le tube 9. Sur le chemin de ce tube 10 est installée une cuve 11 en matière plastique transparente permettant notamment d'évaluer le débit ou la couleur du liquide évacué.

La figure 2 représente une vue en coupe partielle d'un autre mode de réalisation du dispositif d'hémostase selon l'invention. On y distingue les mêmes éléments constitutifs que ceux montrés sur la figure 1. Toutefois, dans ce mode de réalisation, on a un tube 5 servant exclusivement au gonflage du ballon distal 6.

On trouve en plus une double soupape 20 de sortie installée à la partie distale du conduit tubulaire 1, au niveau de la jonction avec le ballon distal 6. Chacun des éléments de cette soupape 20 débouche à l'extérieur du conduit tubulaire 1. Ainsi un fluide de gonflage du ballon distal 6 s'échappera à la partie distale du conduit tubulaire 1 lorsque la pression dans le ballon distal 6 sera supérieure à la valeur prévue. Ce fluide pourra être évacué par les deux tubes 10 de drainage distincts, fermés à leur extrémité distale, dépassant de 5 cm du conduit, formant donc des sortes de tentacules, et comportant chacun une série d'ouvertures 12. Ces deux tubes 10 de drainage arrivent dans une cuve 11 en matière plastique transparente comme on l'a vu ci-dessus. Les deux ballons sont espacés l'un de l'autre de 3 cm selon l'axe du conduit tubulaire 1.

Les éléments de soupape 20 sont un modèle classique à bille et ressort.

Les carrés à l'extrémité proximale des tubes représentent des moyens de connexion optionnels tels que cônes Luer ou Luer-lock, raccords, connecteurs, ou adaptateurs.

La figure 3 représente une vue en coupe selon X-X' de la figure 2. On distingue sur cette figure le conduit tubulaire 1 ainsi que les quatre tubes qui cheminent dans sa lumière. Sur ce modèle, le diamètre externe du conduit tubulaire est de 2,5 cm, le diamètre externe du tube 5 de gonflage du ballon distal 6 est de 0,5 cm, et le diamètre externe des trois autres tubes 8, 10 est de 0,7 cm. L'épaisseur du conduit tubulaire 1 est de 1,5 mm.

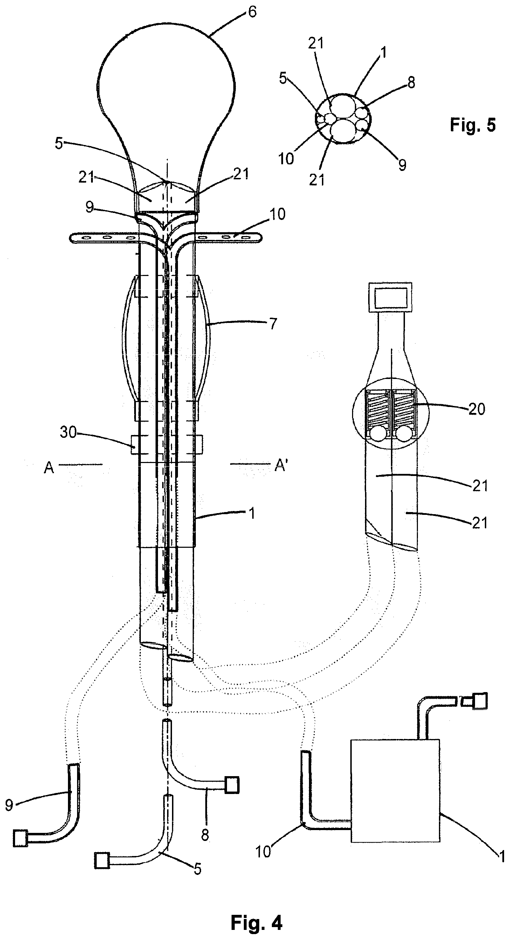

La figure 4 représente une vue en coupe partielle d'un autre mode de réalisation du dispositif d'hémostase ne faisant pas partie de la présente invention. On y distingue les mêmes éléments constitutifs que ceux montrés sur la figure 2. Toutefois, dans ce mode de réalisation, la soupape 20, qui est également double, n'est pas installée dans le conduit, mais vers l'extrémité proximale d'une paire de tubes 21 spécialisés dans l'évacuation d'un liquide de drainage. Chaque soupape est en communication de fluide avec son tube 21 d'évacuation qui débouche du côté distal dans le ballon distal 6. Ainsi, le fluide servant au gonflage du ballon distal 6 est muni de son propre système d'évacuation vers l'extérieur du conduit tubulaire 1. Dans ce mode de réalisation, à la différence de celui de la figure 2, on ne trouve pas deux tubes 10 de drainage, mais un seul tube 10 se terminant en Y du côté distal. Les parties distales du tube 10 de drainage comprennent trois ouvertures: une à l'extrémité distale du tube 10, une en position médiane, et une en position plus proximale

Il est à noter une autre différence. Alors que dans le mode de réalisation de la figure 2 le fluide d'irrigation déversé entre les deux ballons provenait directement des soupapes, dans ce cas, comme dans la figure 1, on trouve un tube 9 servant à irriguer la zone située entre les ballons où il débouche. Comme le tube de drainage 10, il se termine en Y du côté distal.

Un anneau 30 de fixation est installé coulissant à l'extérieur de la partie proximale du conduit tubulaire afin de permettre de fixer le dispositif à une ceinture ou sangle.

La figure 5 représente une vue en coupe selon A-A' de la figure 3. On distingue sur cette figure le conduit tubulaire 1, les quatre tubes qui cheminent dans sa lumière mis en oeuvre dans le mode de réalisation de la figure 2, ainsi que les deux tubes 21 d'évacuation communiquant avec le ballon distal 6. Sur ce modèle, le diamètre externe du conduit tubulaire est de 2,5 cm, le diamètre externe du tube 5 de gonflage du ballon distal 6 est de 0,5 cm, et le diamètre externe des trois autres tubes 8, 10 est encore de 0,7 cm. Le diamètre externe de chacun des tubes 21 d'évacuation est de 1,15 centimètre. L'épaisseur du conduit tubulaire 1 est ici de 2,0 mm.

## Revendications

1. Un dispositif d'hémostase pour un utérus comprenant :
- un conduit tubulaire (1) ayant une extrémité distale (2) et une extrémité proximale (3),
- un ballon distal (6) et un ballon proximal (7)
- trois tubes, d'alimentation ou de récupération de fluide, dont une partie de la longueur est installée dans le conduit tubulaire,
le ballon distal (6) et le ballon proximal étant co-axialement espacés le long du conduit, le ballon distal (6) étant installé à l'extrémité distale du conduit tubulaire, le conduit tubulaire ayant une dimension telle qu'il ne traverse pas le ballon distal une fois gonflé, le ballon distal étant configuré pour être installé et gonflé dans un utérus et épouser la forme de l'ensemble de la cavité utérine traitée à l'état gonflé, le ballon proximal étant installé de manière étanche aux liquides autour d'une portion du conduit tubulaire et étant configuré pour être installé et gonflé dans un vagin,
un (5) des trois tubes débouchant dans le ballon distal (6), un autre (8) des trois tubes débouchant dans le ballon proximal, le troisième des trois tubes (10) débouchant dans un espace extérieur au conduit, situé entre le ballon distal (6) et le ballon proximal (7), **caractérisé en ce que** le ballon distal (6) est en communication de fluide avec au moins une soupape (20) unidirectionnelle pour n'autoriser l'écoulement d'un fluide de gonflage dudit ballon distal (6) que dans le sens de la sortie dudit ballon distal (6), et réglée pour qu'une fois le ballon distal (6) gonflé, toute surpression exercée par la paroi de la cavité corporelle traitée sur la paroi externe du ballon distal (6) entraîne une évacuation de l'excès de fluide de gonflage par au moins une sortie, la ou les soupapes (20) unidirectionnelles étant installées à l'extrémité distale du conduit tubulaire (1) et autorisant le passage de l'excès de fluide vers le ou les sorties débouchant dans un espace extérieur au conduit tubulaire (1), situé entre le ballon distal (6) et le ballon proximal (7).

2. Un dispositif d'hémostase selon la revendication 1, **caractérisé en ce que** le ballon distal (6) et le ballon proximal (7) sont adjacents ou sont longitudinalement espacés par rapport à l'axe du conduit l'un de l'autre de 0,5 à 6,0 cm.

3. Un dispositif d'hémostase selon l'une des revendications 1 et 2, **caractérisé en ce qu'**il comprend un quatrième tube (9, 10) d'alimentation ou de récupération de fluide dont une partie de la longueur est installée dans le conduit tubulaire (1), ce quatrième tube (9, 10) débouchant également dans un espace extérieur au conduit tubulaire (1), situé entre le ballon distal (6) et le ballon proximal (7).

4. Un dispositif d'hémostase selon l'une des revendications 1 à 3, **caractérisé en ce que** le conduit tubulaire (1) a une longueur de 10 à 40 cm.

5. Un dispositif d'hémostase selon l'une des revendications 1 à 4, **caractérisé en ce que** le conduit tubulaire (1) a un diamètre externe de 0,5 à 4 cm.

6. Un dispositif d'hémostase selon l'une des revendications 1 à 5, **caractérisé en ce que** la ou les soupapes (20) sont des soupapes automatiques, dont le seuil de déclenchement est réglé à une valeur allant jusqu'à 200 mm de mercure, avantageusement de 20 à 180 mm de mercure, de préférence de 30 à 130 mm de mercure, notamment de 30 à 120 mm de mercure.

7. Un dispositif d'hémostase selon l'une des revendications 1 à 6, **caractérisé en ce que** le troisième des trois tubes (10) est en communication avec au moins un orifice d'aspiration pour récupérer puis évacuer le liquide formé dans l'espace entre les deux ballons distal et proximal gonflés.

8. Un dispositif d'hémostase selon la revendication 7, **caractérisé en ce que** le ou les orifices d'aspiration sont l'embouchure du troisième tube qui affleure la paroi externe du conduit tubulaire (1), l'embouchure du troisième tube en saillie de la paroi externe du conduit tubulaire (1), ou des orifices formés sur une partie du troisième tube en saillie du conduit tubulaire (1) formant une tubulure multipores.

9. Un dispositif d'hémostase selon l'une des revendications 1 à 8, **caractérisé en ce qu'**un anneau de fixation (30) est installé coulissant à l'extérieur de la partie proximale du conduit tubulaire (1) afin de permettre de fixer le dispositif à une ceinture ou sangle.

10. Un dispositif d'hémostase selon l'une des revendications 1 à 9, **caractérisé en ce qu'**il comprend en outre un fil conducteur d'alimentation en courant et **en ce que** la paroi externe du ballon distal (6) comporte au moins une partie conductrice, laquelle est connectée au fil conducteur d'alimentation en courant, la pression de rupture de la ou des soupapes (20) étant inférieure à une valeur prédéfinie de la pression développée par la pression de l'organe à l'intérieur duquel le ballon distal est destiné à être installé.

## Patentansprüche

1. Hämostasevorrichtung für einen Uterus, umfassend:
- eine rohrförmige Leitung (1), die ein distales Ende (2) und ein proximales Ende (3) aufweist,
- einen distalen Ballon (6) und einen proximalen Ballon (7)
- drei Rohre zur Versorgung oder zur Wiedergewinnung von Fluid, von denen ein Teil der Länge in der rohrförmigen Leitung installiert ist,
wobei der distale Ballon (6) und der proximale Ballon koaxial entlang der Leitung beabstandet sind, wobei der distale Ballon (6) am distalen Ende der rohrförmigen Leitung installiert ist,
wobei die rohrfömige Leitung eine derartige Abmessung aufweist, dass sie den distalen Ballon nicht quert, nachdem sie aufgepumpt ist, wobei der Ballon konfiguriert ist, um in einem Uterus installiert und aufgepumpt zu sein, und um sich der Form der Gesamtheit des behandelten Uterushohlraums im aufgepumpten Zustand anzupassen, wobei der proximale Ballon auf dichte Weise für Flüssigkeiten um einen Abschnitt der rohrförmigen Leitung installiert und konfiguriert ist, um in der Vagina installiert und aufgepumpt zu sein,
wobei eines (5) der drei Rohre in den distalen Ballon (6) mündet, ein anderes (8) der drei Rohre in den proximalen Ballon mündet, das dritte der drei Rohre (10) in einen externen Raum der Leitung mündet, der sich zwischen dem distalen Ballon (6) und dem proximalen Ballon (7) befindet, **dadurch gekennzeichnet, dass** der distale Ballon (6) in fluidischer Kommunikation mit mindestens einem einseitig gerichteten Ventil (20) ist, um den Abfluss eines Aufpumpfluids des distalen Ballons (6) nur in der Richtung des Ausgangs des distalen Ballons (6) zu ermöglichen, und eingestellt dass, nachdem der distale Ballon (6) aufgepumpt ist, jeder Überdruck, der von der Wand des behandelten Körperhohlraums auf die externe Wand des distalen Ballons (6) ausgeübt wird, eine Ableitung des überflüssigen Aufpumpfluids durch mindestens einen Ausgang verursacht, wobei das oder die einseitig ausgerichtete(n) Ventil(e) (20) am distalen Ende der rohrförmigen Leitung (1) installiert ist/sind und den Durchgang des überflüssigen Fluids hin zu dem Ausgang/den Ausgängen, die in einen externen Raum der rohrförmigen Leitung (1) münden, ermöglichen, der sich zwischen dem distalen Ballon (6) und dem proximalen Ballon (7) befindet.

2. Hämostasevorrichtung nach Anspruch 1, **dadurch gekennzeichnet, dass** der distale Ballon (6) und der proximale Ballon (7) benachbart sind oder mit Bezug auf die Achse der Leitung längs von 0,5 bis 6,0 cm voneinander beabstandet sind.

3. Hämostasevorrichtung nach einem der Ansprüche 1 und 2, **dadurch gekennzeichnet, dass** sie ein viertes Rohr (9, 10) zur Versorgung oder zur Wiedergewinnung von Fluid umfasst, von dem ein Teil der Länge in der rohrförmigen Leitung (1) installiert ist, wobei dieses vierte Rohr (9, 10) ebenfalls in einen externen Raum der rohrförmigen Leitung (1) mündet, der sich zwischen dem distalen Ballon (6) und dem proximalen Ballon (7) befindet.

4. Hämostasevorrichtung nach einem der Ansprüche 1 bis 3, **dadurch gekennzeichnet, dass** die rohrförmige Leitung (1) eine Länge von 10 bis 40 cm aufweist.

5. Hämostasevorrichtung nach einem der Ansprüche 1 bis 4, **dadurch gekennzeichnet, dass** die rohrförmige Leitung (1) einen externen Durchmesser von 0,5 bis 4 cm aufweist.

6. Hämostasevorrichtung nach einem der Ansprüche 1 bis 5, **dadurch gekennzeichnet, dass** das oder die Ventil(e) (20) automatische Ventile sind, wobei die Auslöseschwelle auf einen Wert eingestellt ist, der bis 200 mm Quecksilber reicht, vorteilhafterweise von 20 bis 180 mm Quecksilber, vorzugsweise von 30 bis 130 mm Quecksilber, insbesondere von 30 bis 120 mm Quecksilber.

7. Hämostasevorrichtung nach einem der Ansprüche 1 bis 6, **dadurch gekennzeichnet, dass** das dritte der drei Rohre (10) mit mindestens einer Saugöffnung in Kommunikation ist, um das Fluid, gebildet im Raum zwischen den zwei aufgepumpten distalen und proximalen Ballonen wiederzugewinnen und dann abzuleiten.

8. Hämostasevorrichtung nach Anspruch 7, **dadurch gekennzeichnet, dass** die Saugöffnung(en) die Mündung des dritten Rohrs, das aus der externen Wand der rohrförmigen Leitung (1) tritt, die Mündung des dritten Rohrs, das aus der externen Wand der rohrförmigen Leitung (1) hervorspringt, oder Öffnungen sind, die auf einem Teil des dritten Rohrs gebildet sind, das aus der rohrförmigen Leitung (1) hervorspringt, das ein Rohr mit zahlreichen Poren bildet.

9. Hämostasevorrichtung nach einem der Ansprüche 1 bis 8, **dadurch gekennzeichnet, dass** ein Befestigungsring (30) gleitend an der Außenseite des proximalen Teils der rohrförmigen Leitung (1) installiert ist, um zu ermöglichen, die Vorrichtung an einen Gurt oder Riemen zu befestigen.

10. Hämostasevorrichtung nach einem der Ansprüche 1 bis 9, **dadurch gekennzeichnet, dass** sie außerdem einen leitenden Draht zur Versorgung mit Strom umfasst, und dadurch, dass die externe Wand des distalen Ballons (6) mindestens einen leitenden Teil umfasst, der mit dem leitenden Draht zur Versorgung mit Strom verbunden ist, wobei der Bruchdruck des Ventils oder der Ventile (20) geringer als ein vordefinierter Wert des Drucks ist, der vom Druck des Organs entwickelt wird, in dessen Inneren der distale Ballon installiert werden soll.

## Claims

1. A haemostasis device for a uterus comprising :
- a tubular conduit (1) having a distal end (2) and a proximal end (3),
- a distal balloon (6) and a proximal balloon (7),
- three fluid recovery or supply tubes, one part of the length of which is installed in the tubular conduit, wherein the distal balloon (6) and the proximal balloon are coaxially spaced along the conduit, the distal balloon (6) is installed at the distal end of the tubular conduit, wherein the tubular conduit has such a size that it does not pass through the distal balloon once inflated, the distal balloon is configured to be installed and inflated in a uterus and conform to the shape of the entire treated uterine cavity in inflated state, the proximal balloon is liquid-tightly installed around a part of the tubular conduit and configured to be installed and inflated in a vagina,
one (5) of the three tubes opening into the distal balloon (6), another (8) of the three tubes opening into the proximal balloon, the third one of the three tubes (10) opening into a space outside the conduit, located between the distal balloon (6) and the proximal balloon (7), **characterised in that** the distal balloon (6) is in fluid communication with at least one one-way valve (20) for allowing the flow of a fluid for inflating said distal balloon (6) only in the direction of exiting said distal balloon (6), and adjusted such that, once the distal balloon (6) is inflated, any overpressure applied by the wall of the treated body cavity on the outer wall of the distal balloon (6) causes the excess inflating fluid to be discharged through at least one exit, the one or more one-way valves (20) being installed at the distal end of the tubular conduit (1) and allowing the excess fluid to flow to the one or more exits opening into a space outside the tubular conduit (1), located between the distal balloon (6) and the proximal balloon (7).

2. The haemostasis device according to claim 1, **characterised in that** the distal balloon (6) and the proximal balloon (7) are adjacent or longitudinally spaced from each other, with respect to an axis of the conduit, by 0.5-6.0 cm.

3. The haemostasis device according to one of claims 1 and 2, **characterised in that** it comprises a fourth fluid recovery or supply tube (9, 10), one part of the length of which is installed in the tubular conduit (1), this fourth tube (9, 10) also opening into a space outside the tubular conduit (1), located between the distal balloon (6) and the proximal balloon (7).

4. The haemostasis device according to one of claims 1-3, **characterised in that** the tubular conduit (1) has a length from 10 to 40 cm.

5. The haemostasis device according to one of claims 1-4, **characterised in that** the tubular conduit (1) has an outer diameter from 0.5 to 4 cm.

6. The haemostasis device according to one of claims 1-5, **characterised in that** the one or more valves (20) are automatic valves, the trigger threshold of which is adjusted at a value up to 200 mm of mercury, advantageously from 20 to 180 mm of mercury, preferably from 30 to 130 mm of mercury, in particular from 30 to 120 mm of mercury.

7. The haemostasis device according to one of claims 1-6, **characterised in that** the third one of the three tubes (10) is in communication with at least one suction port for recovering and then discharging the liquid formed in the space between both inflated distal and proximal balloons.

8. The haemostasis device according to claim 7, **characterised in that** the one or more suction ports are the mouth of the third tube which is flush with the outer wall of the tubular conduit (1), the mouth of the third tube protrudes from the outer wall of the tubular conduit (1), or ports formed on a part of the third tube protruding from the tubular conduit (1) forming a multipore tubing.

9. The haemostasis device according to one of claims 1-8, **characterised in that** a fastening ring (30) is slidably installed outside the proximal part of the tubular conduit (1) so as to allow the device to be attached to a belt or strap.

10. The haemostasis device according to one of claims 1-9, **characterised in that** it further comprises a current supply lead wire, and **in that** the outer wall of the distal balloon (6) comprises at least one conducting part which is connected to the current supply lead wire, the burst pressure of the one or more valves (20) being lower than a predefined value of the pressure developed by the pressure of the organ in which the distal balloon is intended to be installed.
